# FASCICULE DE BREVET EUROPEEN

(11) **EP 0 659 111 B1**
(45) Date de publication et mention de la délivrance du brevet: **21.05.1997**
(21) Numéro de dépôt: 93919415.5
(22) Date de dépôt: 03.09.1993
(51) Int. Cl.: B29C 45/14, B29C 43/18, B29C 70/00, A61F 2/34

(54) **PROCEDE DE FABRICATION DE PIECES EN MATIERE PLASTIQUE PAR MOULAGE-THERMOFORMAGE CONTROLE**
VERFAHREN ZUR HERSTELLUNG VON KUNSTSTOFFGEGENSTÄNDEN DURCH KONTROLLIERTES FORM-WARMFORMEN
PROCESS FOR THE MANUFACTURE OF PLASTIC PARTS BY CONTROLLED MOULDING-THERMOFORMING

(30) Priorité: 03.09.1992 FR 9210658
(43) Date de publication de la demande: 28.06.1995
(73) Titulaire: LE COENT, Fernand, F-44470 Thouare-sur-Loire (FR)
(72) Inventeur: LE COENT, Fernand, F-44470 Thouare-sur-Loire (FR)
(74) Mandataire: Dawidowicz, Armand
(86) Numéro de dépôt international: FR9300844
(87) Numéro de publication internationale: WO9405481

(56) Documents cités:
- EP-A- 0 041 004
- EP-A- 0 196 946
- EP-A- 0 331 447
- DE-C- 833 118
- FR-A- 2 602 454
- US-A- 4 459 092

## Description

La présente invention concerne un procédé de fabrication par moulage-thermoformage contrôlé de pièces en matière plastique résistant au frottement et au fluage ainsi qu'un dispositif nécessaire à la mise en oeuvre du procédé et les pièces résultant de la mise en oeuvre dudit procédé.

Un certain nombre de pièces en matière plastique telles que les prothèses articulaires ou les pièces mécaniques telles que pignon, plaque de guidage, roulement, palier, et vis, etc. ont une durée de vie limitée dans le temps en raison de phénomènes de fluage de matière et d'usure liés au frottement engendré lors de l'utilisation desdites pièces. Ce problème est particulièrement crucial dans le cas des implants orthopédiques, principalement les prothèses de hanches et de genoux qui sont réalisées en métal avec interposition d'une pièce de frottement. Le polyéthylène à haut poids moléculaire (Pe.h.Pm) s'est notamment imposé pour cette fabrication. Or, ce matériau aux propriétés frottante et anti-usure exceptionnelles ne peut se mettre en oeuvre par les techniques classiques appliquées aux polymères telles que l'injection par exemple. C'est pourquoi la plupart des implants sont réalisés par usinage. Malheureusement, ces réalisations usinées ont le grand inconvénient de ne pas fournir un excellent état de surface présentant des crêtes d'usinage. Or, il s'avère que ces mauvais coefficient et profil de rugosité (Ra) sont à l'origine de débris d'usure dont l'organisme se débarrasse par génération de macrophages qui donnent naissance à des enzymes. Ces derniers s'attaquent ensuite à la prothèse en lysant notamment les matériaux constitutifs de scellement, entraînant ainsi la destruction de la liaison de celle-ci et l'obligation d'une nouvelle intervention chirurgicale.

En conséquence, pour améliorer les propriétés mécaniques de ces pièces soumises à des contraintes mécaniques importantes, il a déjà été proposé un certain nombre de solutions. On connaît ainsi, par FR-A-2.578.780, un procédé qui permet de réaliser des pièces en matière plastique par forgeage en orientant les chaînes moléculaires dudit matériau utilisé, qui est généralement une matière plastique à haut poids moléculaire, et d'améliorer ainsi de manière significative les propriétés mécaniques desdites pièces, en particulier la résistance au fluage. Cependant, le procédé décrit dans ce brevet ne permet pas d'améliorer de manière importante la résitance au fluage, à l'usure et d'obtenir un retrait ou retour élastique de la matière homogène garantissant une fiabilité dimensionnelle. Le manque de résistance à l'usure et au fluage ne permet donc pas d'augmenter notablement la durée de vie des implants grâce à ce procédé.

Un procédé de fabrication de pièces en matière plastique par moulage-thermoformage contrôlé est également décrit dans le brevet US-A-4.459.092. Ce brevet comporte l'introduction dans une cavité de moulage d'une ébauche de matière plastique P1 et d'une matière plastique P2 à une température proche de, ou égale à la température de fusion de P2, la pièce étant formée dans le moule au moyen d'un poinçon mobile dans ladite cavité et de la pression d'injection de la matière plastique P2. L'ébauche P1 ne subit aucun traitement thermique.

Le but de la présente invention est donc de proposer un procédé de fabrication de pièces telles que des prothèses articulaires, des pièces mécaniques diverses, etc, présentant des propriétés mécaniques nettement améliorées notamment en ce qui concerne la résistance au fluage, à la traction, à l'usure et à la fatique sans présenter de retrait important lors de leur fabrication.

Un autre but de l'invention est de proposer un dispositif de mise en oeuvre simple et rapide dudit procédé.

La présente invention concerne à cet effet un procédé de fabrication de pièces en matière plastique par moulage-thermoformage contrôlé selon la revendication 1.

Selon une forme de mise en oeuvre préférée de l'invention, la matière plastique P2 qui présente un poids moléculaire inférieur ou égal à P1 est introduite par injection et on déplace le poinçon pour qu'il vienne en contact de préférence avec l'ébauche de manière à obliger l'ébauche et la matière plastique injectée à épouser le profil de la matrice fermée constituée par les parois de la cavité du moule formant empreinte de la cavité et du poinçon. De ce fait, l'ébauche, qui est constituée d'un matériau qui est un polymère, présente des chaînes moléculaires orientées parallèlement à la surface de frottement au moins dans la zone de la pièce située en-dessous ou affleurant cette surface.

Ce procédé est plus particulièrement destiné à la fabrication de prothèses orthopédiques ou d'implants et de pièces mécaniques diverses.

D'autres caractéristiques et avantages de l'invention apparaîtront encore à la lecture de la description qui suit et des dessins joints, lesquels description et dessins sont donnés surtout à titre d'exemples. Dans ces dessins :
la figure 1 représente une vue en coupe d'un moule équipé d'un poinçon mobile et d'une buse d'injection permettant la mise en oeuvre du procédé de l'invention, ledit moule renfermant dans sa cavité de moulage une ébauche ;
la figure 2 représente le moule de la figure 1 après compression et formage contrôlé de la pièce ;
les figure 3 et 4 représentent des vues en coupe d'un moule de dent de pignon (sans poinçon mobile) respectivement avant compression et après compression ;
la figure 5 représente une vue en coupe d'un moule équipé d'un poinçon mobile avant compression et dont la cavité est remplie de deux ébauches et d'une couche armée stratifiée.

Conformément à l'invention, la mise en oeuvre du procédé permettant la fabrication de pièces en matière plastique qui présentent une bonne résistance à l'usure, au fluage et à la fatigue, nécessite de disposer d'un moule et de réaliser une ébauche 4 de matière plastique P1. Le moule est généralement un moule ouvert en deux parties. Ce moule est généralement équipé d'une buse d'injection et sa cavité de moulage 1 peut être obturée par un poinçon mobile 3. Cette ébauche 4, pour permettre d'optimiser les propriétés mécaniques de la pièce finale, sera de préférence constituée d'un matériau thermoplastique cristallin ou semi-cristallin non injectable tel par exemple qu'une matière plastique à haut poids moléculaire. On peut citer à titre d'exemple le polyéthylène à très haut poids moléculaire qui est fréquemment utilisé notamment dans la réalisation de prothèses articulaires et pièces de frottement. Cette ébauche peut également être renforcée au moyen d'un renfort tissé ou non tissé tel qu'une charge de fibres de verre par exemple. Cette ébauche chauffée présente des zones de température différentes, au moins une des zones étant portée à une température proche ou égale à la température de fusion du matériau P1 utilisé. Par température proche, on entend une température supérieure ou inférieure à la température de fusion de la matière. Par exemple, pour le polyéthylène, on choisit une température comprise dans la plage allant de 20°C en dessous de la température de fusion TF du polyéthylène jusqu'à 10°C au-dessus de cette température de fusion TF ou plus. Il est à noter que la température de fusion TF du polyéthylène est déterminée par calorimétrie et qu'elle correspond au maximum de la vitesse de transformation enregistrée en calorimétrie. Cette ébauche pourra également être chauffée sur une seule face qui sera la face devant adhérer à la matière plastique P2 injectée ou comprimée. On parle dans ce cas de chauffage en superficie ou en surface. Ce chauffage est toujours réalisé préalablement à l'introduction de l'ébauche dans le moule.

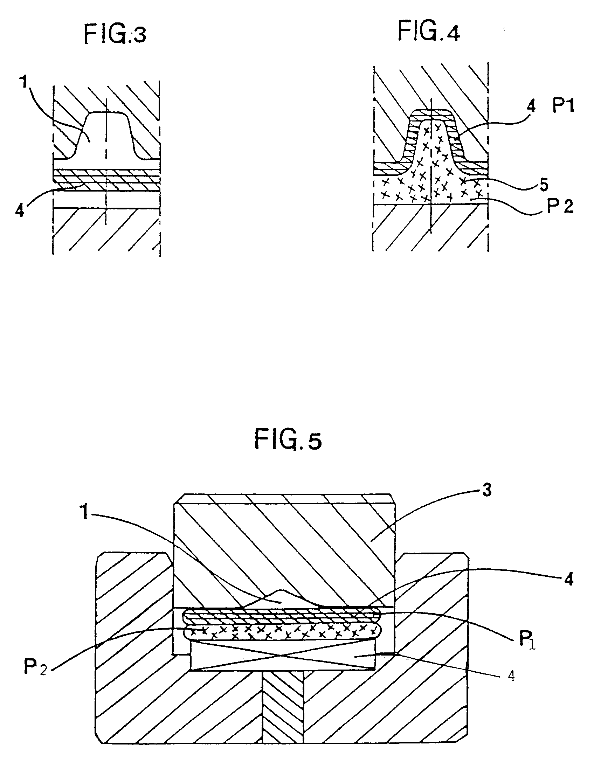

Outre cette ébauche 4, il peut être introduit à l'intérieur de la cavité de moulage une matière plastique P2 portée à une température égale ou proche de la température de fusion, cette matière plastique étant de préférence injectable. Cette matière plastique P2 présente un poids moléculaire inférieur à celui de la matière P1. Cette matière plastique P2 peut être injectée à l'intérieur de la cavité de moulage suivant un grand nombre de modes de réalisation qui sont fonctions de la configuration du moule et du nombre d'ébauches introduites. Ces différents modes de réalisation seront décrits ci-après. On peut également imaginer que la matière plastique P2 ne soit pas, comme c'est le cas souvent pour la matière plastique P1, injectable. Dans ce cas, on dispose les matières plastiques P1 stratifiées et P2 sous formes respectives d'ébauche et de lopin à l'intérieur de la cavité de moulage puis on comprime l'ensemble pour former la pièce. Ce mode de réalisation est représenté à la figure 5.

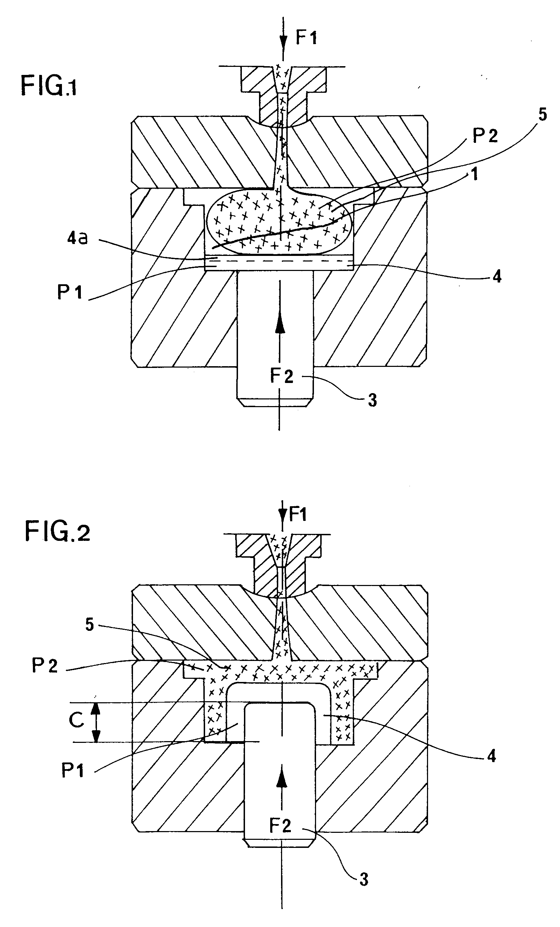

L'étape de compression qui permet le formage de la pièce et qui est subséquente aux étapes d'introduction des matières plastiques P1 et P2 dans le moule peut être réalisée de manière variable et programmée. En effet, lorsque lesdites matières plastiques sont introduites à l'intérieur de la cavité de moulage, on forme la pièce dans le moule soit au moyen de la pression d'injection et uniquement au moyen de celle-ci comme le montre la figure 4, soit au moyen d'un poinçon 3 mobile dans la cavité de moulage comme le montre la figure 5, soit au moyen à la fois de la pression d'injection et du poinçon 3 mobile comme le montre la figure 1. Ces trois moyens pour obtenir une pièce aux propriétés mécaniques intéressantes permettant de couvrir un grand nombre d'applications. En effet, dans certains cas, il est nécessaire que l'ébauche constituée par un matériau hautement résistant au fluage et à la fatigue, c'est-à-dire généralement une matière plastique à haut poids moléculaire et donc pouvant subir un certain nombre de frottements, soit disposée à la surface de la pièce, comme cela est le cas dans les figures 1, 3 et 5. Au contraire, dans certains cas très rares, on préfèrera que ce soit la matière injectable qui soit disposée à la surface de la pièce, comme cela est le cas dans les figures 1, 3 et 5. Au contraire, dans certains cas très rares, on préfèrera que ce soit la matière injectable qui soit disposée en surface de la pièce. Il peut également être intéressant de prendre la matière plastique P2 en sandwich entre deux ébauches ou entre une ébauche et un matériau de renfort ou bien encore de prendre en sandwich entre deux ébauches 4 ou entre l'ébauche et la matière P2 un renfort, ou bien encore d'utiliser l'ébauche 4 seule renforcée avant son introduction dans le moule ou au moyen d'un élément de renfort indépendant. L'intérêt de pouvoir utiliser un poinçon mobile à l'intérieur de la cavité de moulage et d'éventuellement le combiner à la pression d'injection pour former la pièce, comme le montrent les figures 1 et 2, est de pouvoir orienter les chaînes moléculaires du polymère constitutif de l'ébauche de telle sorte qu'elles soient parallèles à la surface de frottement au moins dans la zone de la pièce située en dessous ou affleurant cette surface. Cette caractéristique particulière est notamment obtenue grâce au fait qu'on déplace le poinçon mobile pour qu'il vienne en contact avec l'ébauche 4 de manière à obliger l'ébauche 4 à s'orienter de manière similaire à celle précisée ci-dessus. L'orientation moléculaire consécutive à la mise en oeuvre introduit au niveau des propriétés mécaniques une anisotropie qui peut être préjudiciable à la stabilité dimensionnelle du produit fini. Le fait d'injecter, ou de combiner avec une matière renforcée, une ébauche à basse température augmente l'orientation de celle-ci d'autant plus que la température d'écoulement est faible.

Le procédé permet d'obtenir sur l'ébauche, par exemple la partie frottante de la pièce, des cristallites de faibles tailles et une orientation maximale dans le sens de l'effort. Or, cette orientation peut être mieux contrôlée par le fait que l'on travaille sur un matelas visqueux 5 de la matière injectée P2. L'action du poinçon 3 sur l'ébauche 4 oblige l'ébauche et la matière plastique injectée P2 à épouser le profil de la matrice constituée par les parois de la cavité du moule et éventuellement le poinçon. De plus, le fait de ne réchauffer et porter à température de fusion qu'une partie de l'ébauche comme le montrent par exemple les figures 1 et 2 permet de mieux orienter les chaînes moléculaires dans la partie froide. En outre, ces possibilités de réglage, notamment le fait que l'avance du piston peut être contrôlée par rapport à la valeur de cisaillement de la matière du matelas visqueux ou par rapport à la vitesse d'injection, permettent d'obtenir une pièce exemple de tension interne.

Pour permettre une reprise des efforts mécaniques, il est préférable d'intégrer un renfort armé soit dans l'ébauche 4 soit dans la matière injectée ou non P2. Ainsi, dans le cas de la figure 5, qui représente la fabrication d'une dent de pignon, le matériau renforcé est la matière P2. Au contraire, dans la figure 1, c'est l'ébauche qui est renforcée. Le fait de renforcer l'ébauche permet d'obtenir une pièce dont la surface, à base de matériau à très haut poids moléculaire, possède une meilleure résistance à l'usure, à la fatigue avec un bon coefficient de frottement et avec des performances mécaniques toutefois qui, grâce au renfort, apportent une résistance au fluage, la reprise des efforts se faisant par les matériaux de renfort. Ce matériau de renfort est intégré ou indépendant de l'ébauche.

Il est à noter que le poinçon 3 mobile dans la cavité de moulage 1 est déplaçable en translation dans ladite cavité dans le sens de la hauteur de l'ébauche 4. La pression appliquée au poinçon 3 pour déplacer le poinçon jusqu'à une position finale permettant le formage desdites matières est fonction à la fois de la composition de l'ébauche 4 et éventuellement, lorsqu'elle est présente, de la matière plastique injectée P2. Le poinçon est maintenu en position finale pendant une période variable généralement de quelques secondes de manière à obtenir un retrait homogène et un bon contrôle dimensionnel de la pièce finale sans tension interne. Ensuite, la pièce formée est extraite de la cavité de moulage par des moyens d'éjection en soi connus. Cette cavité de moulage et ce moule peuvent être, en fonction des applications, de forme très variable, le moule pouvant être formé en plusieurs parties, le point d'injection se trouvant sur l'une des parties du moule et le poinçon mobile sur l'une ou l'autre partie du moule ou, au contraire, on peut également utiliser, conformément à la figure 3, un moule comportant uniquement un point d'injection sans poinçon mobile, ou encore un moule comportant uniquement un poinçon mobile. Grâce à ce procédé, il devient aisé de fabriquer des prothèses articulaires, notamment des cotyles de hanche et des plateaux tibiaux ou encore des pièces mécaniques.

Un exemple de fabrication d'un support butée-palier est fourni ci-après conformément aux figures 1 et 2.

L'ébauche 4 en matière plastique P1 est composée d'un polyéthylène à très haut poids moléculaire usiné (6.000.000 de moles). Elle est portée à une température de 160° en surface sur une épaisseur 4a de 2mm, l'autre épaisseur, de 4mm, étant à 80°C environ. La matière injectée 5 en matière plastique P2 est un polyéthylène à très haut poids moléculaire (3.500.000) chargé de fibres de verre. Elle est portée à 170° C. L'ébauche 4 est placée dans le moule puis on injecte très rapidement (1,5 seconde) un volume de matière plus petit que le volume de l'empreinte. Ensuite, le piston comprime l'ébauche sur le matelas de matière injectée jusqu'à la cote C. Pendant cette phase, la pression d'injection est maintenu endessous de la pression résiduelle dans l'empreinte par la force F2 du poinçon. Ensuite, après le refroidissement du canal d'injection, la pression du piston sera programmée dans le temps pendant environ 120 secondes.

L'injection est contrôlée par rapport à l'avance du piston de compression sur l'ébauche, ce qui permet d'ajuster les taux d'orientation de la matière de l'ébauche.

La partie côté d'injection du moule sera régulée à 50-60°C, celle côté compression ébauche sera régulée de 125°C à 60°C linéairement pendant un temps de 240 secondes. La pression exercée sur le piston sera également dégressive de 120kg/cm² à 50 Kg/cm² pendant le temps de refroidissement de 240 secondes suivant une courbe appropriée.

Cela évitera toute contrainte dans la pièce moulée et permettra d'ajuster les phases amorphes et cristallines.

Il est à noter que ces valeurs numériques ne sont fournies qu'à titre d'exemples.

## Revendications

1. Procédé de fabrication de pièces en matière plastique par moulage-thermoformage contrôlé, du type comportant l'introduction dans une cavité de moulage (1) d'au moins une ébauche (4) de matière plastique P1 et, facultativement, l'introduction dans ladite cavité de moulage (1) d'une matière plastique P2 à une température proche de, ou égale à, la température de fusion de la matière plastique P2, selon lequel on chauffe préalablement ladite ébauche de manière à ce qu'elle présente des zones de température différentes, au moins l'une des zones de température étant proche de ou égale à la température de fusion de la matière plastique P1, et selon lequel on forme la pièce dans le moule au moyen d'un poinçon (3) mobile dans la cavité de moulage (1) et/ou de la pression d'injection de la matière plastique P2 lorsque celle-ci est injectée.

2. Procédé de fabrication selon la revendication 1,
caractérisé en ce que l'ébauche (4) de matière plastique P1 présente au moins une zone de température proche de, ou égale à la température de fusion de P1.

3. Procédé de fabrication selon la revendication 1,
caractérisé en ce qu'on renforce la matière plastique P1 préalablement à son introduction dans la cavité (1) de moulage au moyen d'un matériau de renfort approprié.

4. Procédé de fabrication selon la revendication 1,
caractérisé en ce qu'on introduit un élément de renfort indépendant antérieurement ou postérieurement à l'introduction de la matière plastique P1 dans la cavité de moulage.

5. Procédé selon la revendication 1,
caractérisé en ce que l'ébauche (4) est constituée d'au moins un matériau thermoplastique P1 non injectable tel qu'une matière plastique à très haut poids moléculaire.

6. Procédé selon la revendication 5,
caractérisé en ce que l'ébauche (4) est un polymère dont les chaînes moléculaires sont orientées parallèlement à la surface de frottement au moins dans la zone de la pièce située en dessous ou affleurant cette surface.

7. Procédé selon l'une des revendications 1, 3 ou 4,
caractérisé en ce qu'on renforce la matière plastique P2.

8. Procédé selon l'une des revendications 3, 4 ou 7,
caractérisé en ce que le renfort est constitué par un matériau tissé.

9. Procédé selon la revendication 1,
caractérisé en ce qu'on déplace le poinçon (3) pour qu'il vienne en contact de préférence avec l'ébauche (4) de manière à obliger l'ébauche (4) à épouser le profil de la matrice fermée constituée par les parois de la cavité de moulage (1) et du poinçon (3).

10. Procédé selon la revendication 1,
caractérisé en ce qu'on injecte la matière plastique P2 pour qu'elle vienne en contact de préférence avec l'ébauche (4) de manière à obliger l'ébauche à épouser le profil de la matrice fermée constituée par les parois de la cavité de moulage (1) et du poinçon (3) en position rentrée dans ladite cavité (1).

11. Procédé selon l'une des revendications précédentes,
caractérisé en ce que, sous l'action de la pression d'injection de la matière plastique P2 et/ou du poinçon mobile (3), on met au moins la matière plastique P1 à la forme d'un implant.

12. Procédé selon la revendication 11,
caractérisé en ce que ledit implant est un cotyle.

13. Procédé selon l'une des revendications 1 à 10,
caractérisé en ce que, sous l'action de la pression d'injection de la matière plastique P2 et/ou du poinçon mobile, on met au moins la matière plastique P1 à la forme d'une pièce mécanique.

## Claims

1. A process for manufacturing parts of plastics material by moulding-thermoforming, of the type comprising introduction of at least one preform (4) of plastics material P1 into a moulding cavity (1) and optionally introduction into the said moulding cavity (1) of plastics material P2 at a temperature close to or equal to the melting point of the plastics material P2, according to which the said preform is first heated in such a manner that it has zones at different temperatures, at least one of the temperature zones being close to or equal to the melting point of the plastics material P1, and according to which the part is shaped in the mould by means of a die (3) movable into the moulding cavity (1) and/or of the pressure of injection of the plastics material P2 when this is injected.

2. A manufacturing process according to claim 1, characterized in that the preform (4) of plastics material P1 has at least one temperature zone close to or equal to the melting point of P1.

3. A manufacturing process according to claim 1, characterized in that the plastics material P1 is reinforced by means of a suitable reinforcing material before it is introduced into the moulding cavity (1).

4. A manufacturing process according to claim 1, characterized in that an independent reinforcing element is introduced before or after the introduction of the plastics material P1 into the moulding cavity.

5. A process according to claim 1, characterized in that the preform (4) is formed from at least one non-injectable thermoplastic material P1, such as a plastics material of very high molecular weight.

6. A process according to claim 5, characterized in that the preform (4) is a polymer whose chains of molecules are orientated parallel to the rubbing surface at least in the zone of the part beneath or flush with this surface.

7. A process according to claim 1, 3 or 4, characterized in that the plastics material P2 is reinforced.

8. A process according to claim 3, 4 or 7, characterized in that the reinforcement is formed by a woven material.

9. A process according to claim 1, characterized in that the die (3) is displaced until it comes into contact preferably with the preform (4) in such a manner as to force the preform (4) to assume the profile of the closed matrix formed by the walls of the moulding cavity (1) and of the die (3).

10. A process according to claim 1, characterized in that the plastics material P2 is injected until it comes into contact preferably with the preform (4) in such a manner as to force the preform to assume the profile of the closed matrix formed by the walls of the moulding cavity (1) and of the die (3) in a position entering the said cavity (1).

11. A process according to any of the preceding claims, characterized in that at least the plastics material P1 is put into the shape of an implant under the action of the injection pressure of the plastics material P2 and/or of the movable die (3).

12. A process according to claim 11, characterized in that the said implant is a cotyle.

13. A process according to any of claims 1 to 10, characterized in that at least the plastics material P1 is put into the shape of mechanical part under the action of the injection pressure of the plastics material P2 and/or of the movable die.

## Patentansprüche

1. Verfahren zur Herstellung von Kunststoffgegenständen durch kontrolliertes Form-Warmformen mit der Einführung mindestens eines Vorformlings (4) aus Kunststoff P1 in einen Formraum (1) und fakultativ mit der Einführung eines Kunststoffs P2 mit einer Temperatur nahe oder gleich der Schmelztemperatur des Kunststoffs P2 in den besagten Formraum, nach dem zuvor der besagte Vorformling so erhitzt wird, daß er unterschiedliche Temperaturbereiche aufweist, wobei mindestens einer der Temperaturbereiche nahe oder gleich der Schmelztemperatur des Kunststoffs P1 ist, und nach dem das Formen des Gegenstands im Formwerkzeug mittels eines im Formraum (1) beweglichen Stempels (3) und/oder des Spritzdrucks des Kunststoffs P2, wenn dieser eingespritzt wird, erfolgt.

2. Herstellungsverfahren nach Anspruch 1, **dadurch gekennzeichnet,** daß der Vorformling (4) aus Kunststoff P1 mindestens einen Temperaturbereich nahe oder gleich der Schmelztemperatur von P1 aufweist.

3. Herstellungsverfahren nach Anspruch 1, **dadurch gekennzeichnet,** daß der Kunststoff P1 vor seiner Einführung in den Formraum (1) mittels eines geeigneten Verstärkungswerkstoffs verstärkt wird.

4. Herstellungsverfahren nach Anspruch 1, **dadurch gekennzeichnet,** daß ein unabhängiges Verstärkungselement vor oder nach der Einführung des Kunststoffs P1 in den Formraum eingeführt wird.

5. Verfahren nach Anspruch 1 , **dadurch gekennzeichnet,** daß der Vorformling (4) aus mindestens einem nicht spritzfähigen Thermoplast P1 besteht, etwa aus einem Kunststoff mit sehr hohem Molekulargewicht.

6. Verfahren nach Anspruch 5 , **dadurch gekennzeichnet,** daß der Vorformling (4) ein Polymer ist, dessen Molekülketten parallel zur Reibungsfläche zumindest in dem Bereich des Gegenstands orientiert sind, der unterhalb dieser Fläche oder daran anliegend angeordnet ist.

7. Verfahren nach einem der Ansprüche 1, 3 oder 4, **dadurch gekennzeichnet,** daß der Kunststoff P2 verstärkt wird.

8. Verfahren nach einem der Ansprüche 3, 4 oder 7, **dadurch gekennzeichnet,** daß die Verstärkung aus einem Gewebematerial besteht.

9. Verfahren nach Anspruch 1 , **dadurch gekennzeichnet,** daß der Stempel (3) so verschoben wird, daß er vorzugsweise mit dem Vorformling (4) in Berührung kommt, damit sich der Vorformling (4) an das Profil der geschlossenen Matrize anpassen muß, die durch die Wände des Formraums (1) und des Stempels (3) gebildet wird.

10. Verfahren nach Anspruch 1 , **dadurch gekennzeichnet,** daß der Kunststoff P2 so eingespritzt wird, daß er vorzugsweise mit dem Vorformling (4) in Berührung kommt, damit sich der Vorformling an das Profil der geschlossenen Matrize anpassen muß, die durch die Wände des Formraums (1) und des Stempels (3) in Zurückgefahrener Position in dem besagten Formraum (1) gebildet wird.

11. Verfahren nach einem der vorangehenden Ansprüche, **dadurch gekennzeichnet,** daß unter der Einwirkung des Spritzdrucks des Kunststoffs P2 und/oder des beweglichen Stempels (3) zumindest der Kunststoff P1 in die Form eines Implantats gebracht wird.

12. Verfahren nach Anspruch 11 , **dadurch gekennzeichnet,** daß das besagte Implantat eine Hüftgelenkpfanne ist.

13. Verfahren nach einem der Ansprüche 1 bis 10, **dadurch gekennzeichnet,** daß unter der Einwirkung des Spritzdrucks des Kunststoffs P2 und/oder des beweglichen Stempels zumindest der Kunststoff P1 in die Form eines Maschinenteils gebracht wird.
